# EUROPÄISCHE PATENTSCHRIFT

(11) **EP 1 253 938 B1**
(45) Veröffentlichungstag und Bekanntmachung des Hinweises auf die Patenterteilung: **25.04.2007**
(21) Anmeldenummer: 01913586.2
(22) Anmeldetag: 07.02.2001
(51) Int. Cl.: A61K 39/00, A61P 35/00

(54) **Immunisierung gegen Karzinome und ihre Vorstufen**
Immunization against carcinoma and the preliminary stages thereof
Immunisation contre des carcinomes et leurs stades préalables

(30) Priorität: 10.02.2000 DE 10006033
(43) Veröffentlichungstag der Anmeldung: 06.11.2002
(73) Patentinhaber: Von Knebel Doeberitz, Magnus, 69120 Heidelberg (DE)
(72) Erfinder: VON KNEBEL DOEBERITZ, Magnus, 69120 Heidelberg (DE); LINNEBACHER, Michael, 66578 Stennweiler (DE); RUDY, Wolfgang, 75015 Bretten (DE)
(74) Vertreter: Schüssler, Andrea
(86) Internationale Anmeldenummer: PCT/DE2001/000470
(87) Internationale Veröffentlichungsnummer: WO 2001/058477

(56) Entgegenhaltungen:
- WO-A-98/45444
- WO-A-99/02183
- WO-A-99/19357
- DE-A- 19 829 473
- CARTER, J. H. (1) ET AL: "Cyclin - an overexpressed cell cycle protein as a potential tumour antigen target for immunotherapy." IMMUNOLOGY, (DEC., 1998) VOL. 95, NO. SUPPL. 1, PP. 104. MEETING INFO.: 6TH ANNUAL CONGRESS OF THE BRITISH SOCIETY FOR IMMUNOLOGY HARROGATE, ENGLAND, UK DECEMBER 1-4, 1998 , XP002177603

## Beschreibung

Die vorliegende Erfindung betrifft die Verwendung einer pharmazeutischen Zusammensetzung zur Immunisierung eines Individuums gegen Carcinome und ihre Vorstufen.

An Carcinomen erkranken und versterben jährlich mehrere Millionen Menschen weltweit. Diese Sterblichkeitsraten sind seit vielen Jahren unverändert trotz intensiver Therapie-Forschungen. Bisher werden die Patienten mit Carcinomen vielfach einer chirurgischen Entfernung der Carcinome bzw. einer Chemo- oder Strahlentherapie unterzogen. Damit sind aber massivste Nebenwirkungen verbunden, die dann zu den Sterblichkeitsraten der Patienten mit Carcinomen beitragen.

Der vorliegenden Erfindung liegt somit die Aufgabe zugrunde, ein Mittel bereitzustellen, mit dem gegen Carcinome therapeutisch und prophylaktisch vorgegangen werden kann, wobei vorstehende Nebenwirkungen vermieden werden.

Erfindungsgemäß wird dies durch die Gegenstände in den Patentansprüchen erreicht.

Die vorliegende Erfindung beruht auf den Erkenntnissen des Anmelders, daß bei Carcinomen bzw. ihren Vorstufen Zellzyklus-Regulatorproteine in veränderter Form oder Menge vorliegen. Beispielsweise findet sich in Carcinomen eine Überexpression von Zyklin-abhängigen Kinase-Inhibitoren (vgl. deutsches Patent 198 29 473 des Anmelders). Ferner hat der Anmelder erkannt, daß Individuen gegen in Form oder Menge veränderte Zellzyklus-Regulatorproteine immunisiert werden können, wodurch gegen Carcinome und ihre Vorstufen therapeutisch und prophylaktisch vorgegangen werden kann. Der Anmelder hat dies in invitro- wie auch in invivo-Experimenten gezeigt (vgl. nachstehendes Beispiel).

Somit betrifft die vorliegende Erfindung die Verwendung zur Herstellung einer pharmazeutischen Zusammensetzung, umfassend das Zellzyklus-Regulatorprotein p16 und/oder eine exprimierbare hierfür kodierende Nukleinsäure gemäss Anspruch 1.

Die Zellzyklus-Regulatorproteine können in Wildtyp- oder veränderter Form vorliegen. Letztere Form umfaßt Veränderungen der Aminosäuresequenz, wie Additionen, Deletionen, Substitutionen und/oder Inversionen von einer oder mehreren Aminosäuren. Auch können Fragmente von Zellzyklus-Regulatorproteinen als solche oder in Verbindung mit Trägern vorliegen, wobei die Fragmente eine Wildtyp- oder eine veränderte Aminosäuresequenz haben können. Günstig ist es, wenn die Träger im Individuum nicht als immunogen wirken. Solche Träger können Individuum-eigene oder -fremde Proteine bzw. Fragmente davon sein. Bevorzugt sind Träger, wie Serumalbumin, Fibrinogen oder Transferrin bzw. ein Fragment davon. Besonders günstig ist es, wenn die Fragmente der Zellzyklus-Regulatorproteine Epitope enthalten, die von cytotoxischen T-Zellen, z.B. CD8⁺ T-Zellen, erkannt werden und eine cytotoxische Immunantwort induzieren können. Solche Epitope von Zellzyklus-Regulatorproteinen können durch dem Fachmann bekannte Verfahren, insbesondere durch Verwendung eines Softwaresystems des NIH (NIH bioinformation service http:/bimas.dcrt.nih.gov.cgi-bin/molbio/ken_parker_comboform) ermittelt werden. Von Vorteil kann es ferner sein, wenn unterschiedliche Zellzyklus-Regulatorproteine oder Fragmente davon, für die vorstehenden Ausführungen entsprechend gelten, gleichzeitig vorliegen. Zur Herstellung vorstehender Zellzyklus-Regulatorproteine wird z.B. auf Sambrook et al., Molecular Cloning: A Laboratory Manual, 2. Ausgabe, Cold Spring Harbor Laboratory Press, Cold Spring Harbor NY (1989) verwiesen.

Der verwendete Ausdruck "exprimierbare für ein Zellzyklus-Regulatorprotein kodierene Nukleinsäure" umfaßt jegliche Nukleinsäure, z.B. RNA oder DNA, die in einem Individuum exprimierbar ist und für ein Zellzyklus-Regulatorprotein, für das vorstehende Ausführungen entsprechend gelten, kodiert. Die Nukleinsäure kann als solche, d.h. zusammen mit für ihre Expression geeigneten Elementen, oder in Verbindung mit einem Vektor vorliegen. Beispiele solcher Elemente sind Promotoren und Enhancer, wie CMV-, SV40-, RSV-, Metallothionein I und Polyhedrin-Promotor bzw. CMV- und SV40-Enhancer. Weitere für die Expression geeignete Sequenzen gehen aus Goeddel: Gene Expression Technology: Methods in Enzymology 185, Academic Press, San Diego, CA (1990) hervor. Darüberhinaus können als Vektoren jegliche für die Expression in Säugerzellen geeignete Vektoren verwendet werden. Dies sind z.B. pcDNA3, pMSX, pKCR, pEFBOS, cDM8 und pCEV4 sowie von pcDNAI/amp, pcDNAI/neo, pRc/CMV, pSV2gpt, pSV2neo, pSV2-dhfr, pTk2, pRSVneo, pMSG, pSVT7, pko-neo und pHyg stammende Vektoren. Auch können als Vektoren rekombinante Viren, z.B. Adenovirus, Vaccinia-Virus oder Adeno-assoziiertes Virus, verwendet werden. Hinsichtlich der Herstellung vorstehender Nukleinsäuren, insbesondere von Vektoren, die solche Nukleinsäuren enthalten, wird z.B. auf Sambrook et al., supra, verwiesen.

Der verwendete Ausdruck "Carcinome und ihre Vorstufen" umfaßt Carcinome des oberen Respirationstraktes oder Anogenital-Carcinome, insbesondere das Cervix-Carcinom und seine Vorstufen, wie cervikale intraepitheliale Neoplasie (CIN I-III), Carcinoma in situ (CIS), etc. sein.

Der verwendete Ausdruck "Individuum" umfaßt ein Individuum jeglicher Art und Herkunft, das Zellzyklus-Regulatorproteine aufweist und an Carcinomen bzw. ihren Vorstufen erkranken kann. Beispiele eines solchen Individuums sind der Mensch und das Tier sowie Zellen von diesen.

Der verwendete Ausdruck "für eine Immunisierung eines Individuums geeignete Menge" umfaßt jegliche Menge eines Zellzyklus-Regulatorproteins, für das vorstehende Ausführungen entsprechend gelten, bzw. einer exprimierbaren hierfür kodierenden Nukleinsäure, für die vorstehende Ausführungen entsprechend gelten, mit der ein Individuum immunisiert werden kann. Die Menge hängt davon ab, ob ein Zellzyklus-Regulatorprotein oder eine exprimierbare hierfür kodierende Nukleinsäure verwendet wird. Auch hängt die Menge davon ab, ob die Immunisierung des Individuums mehr auf eine Induktion von gegen veränderte Zellzyklus-Regulatorproteine gerichteten Antikörpern oder auf eine Stimulierung von gegen veränderte Zellzyklus-Regulatorproteine gerichteten cytotoxischen T-Zellen, z.B. CD8⁺ T-Zellen, abzielt. Beide Möglichkeiten der Immunisierung können durch die vorliegende Erfindung erreicht werden. Desweiteren hängt die Menge davon ab, ob die Immunisierung als prophylaktische oder therapeutische Behandlung beabsichtigt ist. Darüber hinaus spricht das Alter, das Geschlecht und das Gewicht des Individuums eine Rolle für die Bestimmung der Menge. Günstig ist es, wenn dem Individuum 100µg - 1 g eines Zellzyklus-Regulatorproteins bzw. 10⁶ - 10¹² MOI eines rekombinanten, eine exprimierbare für ein Zellzyklus-Regulatorprotein kodierende Nukleinsäure enthaltenden Virus injiziert werden. Die Injektion kann an mehreren Stellen des Individuums intramuskulär, subkutan, intradermal oder in jeder anderen Applikationsform erfolgen. Ferner kann es günstig sein, ein oder mehrere "Booster-Injektionen" mit ca. gleicher Menge durchzuführen, wobei es besonders günstig sein kann, in den einzelnen Injektionen verschiedene Fragmente des p16 Proteins zu verwenden.

Der verwendete Ausdruck "übliche Hilfsstoffe" umfaßt jegliche Hilfsstoffe, die sich für eine pharmazeutische Zusammensetzung zur Immunisierung eines Individuums eignen. Solche Hilfsstoffe sind z.B. Immunisierungs-Adjuvantien, wie GM-CSF oder Freund's Adjuvans, gepufferte Kochsalzlösungen, Wasser, Emulsionen, wie Öl/Wasser-Emulsionen, Netzmittel, sterile Lösungen, etc.

Mit der vorliegenden Erfindung ist es möglich, Individuen, insbesondere den Menschen und Tiere, gegen veränderte Zellzyklus-Regulatorproteine zu immunisieren. Die Immunisierung erfolgt sowohl durch die Induktion von Antikörpern als auch durch die Stimulierung von CD8⁺-T Zellen, die gegen veränderte Zellzyklus-Regulatorproteine gerichtet sind. Somit ist ein prophylaktisches und therapeutisches Vorgehen gegen Carcinome und ihre Vorstufen möglich.

Die Erfindung wird durch das nachfolgende Beispiel erläutert.

### Beispiel: Stimulierung von CD8⁺-T Zellen gegen den Zyklinabhänigen Kinase-Inhibitor p16 und Lyse von p16-überexprimierenden Carcinom-Zellen.

### (A) Stimulierung von CD8⁺-T Zellen gegen p16.

Von einem gesunden Spender werden periphere mononukleäre Zellen gewonnen und einer sog. ELISPOT-Analyse unterzogen. Das Prinzip dieses Experimentes ist, daß Lymphozyten in Kulturgefäßen mit spezifischen Antigenen stimuliert werden. Kommt es zur Aktivierung der Lymphozyten, da diese das Antigen erkennen, setzen die aktivierten Lymphozyten Zytokine frei, die ihrerseits an spezifische Antikörper binden, welche auf der Bodenfläche der Kulturgefäße immobilisiert sind. Nach dem Auswaschen der Lymphozyten können dann die gebundenen Zytokine in den Kulturgefäßen mit Hilfe eines zweiten Antikörpers, der in einer nachgeschalteten Farbreaktion sichtbar gemacht wird, nachgewiesen werden.

Periphere Blutlymphozyten (PBL) werden von einem HLA-A0201 positiven gesunden Probanden durch Dichtezentrifugation über einen Ficoll Paque^{®}-Gradienten aufgereinigt. T-Lymphozyten werden durch Abtrennung der B-Lymphozyten bzw. der Monozyten mit Hilfe Antikörper gekoppelter Magnetobeads (CD11, CD16, CD19, CD36 und CD56) (Pant T cell isolation Kit^{®}, Milteny, Bergisch Gladbach, Germany) gewonnen. Aus 30 ml Blut werden etwa 2 x 10⁷ T-Zellen gewonnen werden.

HLA-A0201 restringierte Peptide von p16 werden mittels eines Softwaresystems des NIH (NIH bioinformation service [http:/bimas.dcrt.nih.gov.cgi-bin/molbio/ken_parker_comboform) identifiziert. Es handelt sich um die nachstehenden Peptide:

| **9mer Peptide:** | **10mer Peptide:** |
|---|---|
| score 1: VMMMGSARV | score 1: MMGSARVAEL |
| score 2: VLHRAGARL | score 2: LLLHGAEPNC |
| score 3: TLTRPVHDA | score 3: GVMMMGSARV |
| score 4: LLHGAEPNC | |
| score 5: SMEPSADML | |

Die isolierten T-Zellen werden mit T2-Zellen inkubiert, die (a) mit einem Gemisch der vorstehenden 9mer Peptide (10µg/Peptid) und (b) mit einem Gemisch der vorstehenden 10mer Peptide (10µg/Peptid) beladen worden sind. Die T-Zellen werden über 6-Wochen jeweils wöchentlich restimuliert. Jeweils 10⁷ T-Zellen werden mit 2 x 10⁶ Peptid-beladenen T2-Zellen in 24 Lochplatten cokultiviert.

Die Reaktivität gegenüber den Peptid-beladenen T2-Zellen wird wöchentlich bestimmt, beginnend am Tag 0 des Experiments, indem eine IFN-γ Elispotanalyse durchgeführt wird. Am Tag 28 wird eine Reaktivität durch das Gemisch von (a) (400 spezifische Zellen pro Million Zellen) beobachtet. Die Hauptreaktivität ist dabei gegen das Peptid VMMMGSARV (1000 spezifische Zellen / 1 000 000 Zellen) gerichtet (Fig. 1). Eine schwächere Aktivität wird gegen das Gemisch von (b) (150 spezifische Zellen / 1 000 000 Zellen) beobachtet. Hier zeigt das Peptid MMGSARVAEL die höchste Reaktivität (600 spezifische Zellen / 1 000 000 Zellen).

Somit wird deutlich, daß gegen p16 aktivierte CD8⁺-T Zellen stimuliert werden können.

### (B) Lyse von p16-überexprimierenden Carcinom-Zellen

Nach einer weiteren Restimulierung werden die aktivierten CD8⁺-T Zellen mit den HLA A0201+ Zervixkarzinomzellen Caski, die p16 überexprimieren, inkubiert. Als Kontrollen werden die Kolonkarzinomzellen SW480 verwendet, die p16 nicht überexprimieren. 10⁶ Caski-Zellen werden mit ⁵¹Cr (100µCi) für 1 h bei 37°C markiert und mit steigenden Zahlen an aktivierten CD8⁺-T Zellen für 3 Stunden kokultiviert. Die spezifische Lyse der Caski-Zellen wird durch die Menge der freigesetzten Radioaktivität im Überstand bestimmt.

Es zeigt sich, daß Caski-Zellen durch die aktivierenden CD8⁺-T Zellen, nicht aber die Kontrollzellen SW480 lysiert werden (Fig. 2).

## Patentansprüche

1. Verwendung eines Zellzyklus-Regulatorproteins als ganzes oder in der Form eines oder mehrerer immunogener Fragmente des Proteins und/oder einer exprimierbaren hierfür kodierenden Nukleinsäure zur Herstelllung eines Arzneimittels zur Immunisierung eines Individuums gegen Carcinome und ihre Vorstufen, wobei das Zellzyklus-Regulatorprotein der Zyklin-abhängige Kinase-Inhibitor p16 ist und die Carcinome solche des oberen Respirationstraktes bzw. Anogenital-Carcinome sind.

2. Verwendung nach Anspruch 1, wobei das Zellzyklus-Regulatorprotein in Form eines oder mehrerer immunogener Fragmente vorliegt.

3. Verwendung nach Anspruch 2, wobei das oder die Fragmente von cytotoxischen T-Zellen erkennbare und eine cytotoxische Immunantwort auslösende Epitope enthalten.

4. Verwendung nach einem der Ansprüche 1 bis 3, wobei das Anogenital-Carcinom ein Cervix-Carcinom ist.

5. Verwendung nach Anspruch 1, wobei das Zellzyklus-Regulatorprotein ein Fragment des Zyklin-abhängigen Kinase-Inhibitors p16 ist und die Sequenz VMMMGSARV, VLHRAGARL, TLTRPVHDA, LLHGAEPNC, MMGSARVAEL, LLLHGAEPNC oder GVMMMGSARV aufweist.

6. Verwendung nach Anspruch 5, wobei das Anogenital-Carcinom ein Cervix-Carcinom ist.

## Claims

1. Use of a cell cycle regulatory protein as a whole or in the form of one or more immunogenic fragments of the protein and/or an expressible nucleic acid coding therefor for the production of a medicament for immunizing an individual against carcinomas and their preliminary stages, the cell cycle regulatory protein being the cyclin-dependent kinase inhibitor p16 and the carcinomas being those of the upper respiratory tract and/or anogenital carcinomas.

2. Use according to claim 1, wherein the cell cycle regulatory protein is available in the form of one or more immunogenic fragments.

3. Use according to claim 2, wherein the fragment or fragments of cytotoxic T-cells contain detectable epitopes triggering a cytotoxic immune response.

4. Use according to any of claims 1 to 3, wherein the anogenital carcinoma is a cervical carcinoma.

5. Use according to claim 1, wherein the cell cycle regulatory protein is a fragment of the cyclin-dependent kinase inhibitor p16 and the sequence includes VMMMGSARV, VLHRAGARL, TLTRPVHDA, LLHGAEPNC, MMGSARVAEL, LLLHGAEPNC or GVMMMGSARV.

6. Use according to claim 5, wherein the anogenital carcinoma is a cervical carcinoma.

## Revendications

1. Utilisation d'une protéine régulatrice de cycle de cellule sous la forme d'une protéine complète ou bien sous la forme d'un ou plusieurs fragments immunogènes de protéine et/ou d'un acide nucléique exprimable codant à cette fin pour la préparation d'un médicament destiné à l'immunisation d'un individu contre le carcinome et ses stades précurseurs, **caractérisée en ce que** la protéine régulatrice de cycle de cellule est constituée par l'inhibiteur p16 kinase dépendant de la cycline et les carcinomes sont ceux du tractus respiratoire supérieur ou des carcinomes anogénitaux.

2. Utilisation selon la revendication 1, **caractérisée en ce que** la protéine régulatrice de cycle de cellule se présente sous la forme d'un ou plusieurs fragments immunogènes.

3. Utilisation selon la revendication 2, **caractérisé en ce que** le ou les fragments sont reconnaissables par les cellules T cytotoxiques et renferment un épitope déclenchant une réponse immune cytotoxique.

4. Utilisation selon l'une des revendications 1 à 3, **caractérisée en ce que** le carcinome anogénital est un carcinome cervix.

5. Utilisation selon la revendication 1, **caractérisée en ce que** la protéine régulatrice de cycle de cellule est un fragment de l'inhibiteur p16 kinase dépendant de la cycline et la séquence comprend VMMMGSARV, VLHRAGARL, TLTRPVHDA, LLHGAEPNC, MMGSARVAEL, LLLHGAEPNC ou GVMMMGSARV.

6. Utilisation selon la revendication 5, **caractérisée en ce que** le carcinome anogénital est un carcinome cervix.


### SEQUENZPROTOKOLL

<110> von Knebel-Doeberitz, Magnus
   Linnebacher, Michael
   Rudy, Wolfgang
<120> Immunisierung eines Individuums gegen Carcinome und ihre Vorstufen
<130> K2935
<140> PCT/DE01/00470
   <141> 2001-02-07
<150> DE 100 06 033.1
   <151> 2000-02-10
<160> 8
<170> PatentIn Ver. 2.1
<210> 1
   <211> 9
   <212> PRT
   <213> Künstliche Sequenz
<220>
   <223> Beschreibung der künstlichen Sequenz: HLA-A0201 restringiertes Peptid von p16
<400> 1
   Val Met Met Met Gly Ser Ala Arg Val
     1               5
<210> 2
   <211> 9
   <212> PRT
   <213> Künstliche Sequenz
<220>
   <223> Beschreibung der künstlichen Sequenz: HLA-A0201 restringiertes Peptid von p16
<400> 2
   Val Leu His Arg Ala Gly Ala Arg Leu
   1 5
<210> 3
   <211> 9
   <212> PRT
   <213> Künstliche Sequenz
<220>
   <223> Beschreibung der künstlichen Sequenz: HLA-A0201 restringiertes Peptid von p16
<400> 3
   Thr Leu Thr Arg Pro Val His Asp Ala
   1 5
<210> 4
   <211> 9
   <212> PRT
   <213> Künstliche Sequenz
<220>
   <223> Beschreibung der künstlichen Sequenz: HLA-A0201 restringiertes Peptid von p16
<400> 4
   Leu Leu His Gly Ala Glu Pro Asn Cys
   1 5
<210> 5
   <211> 9
   <212> PRT
   <213> Künstliche Sequenz
<220>
   <223> Beschreibung der künstlichen Sequenz: HLA-A0201 restringiertes Peptid von p16
<400> 5
   Ser Met Glu Pro Ser Ala Asp Met Leu
   1 5
<210> 6
   <211> 10
   <212> PRT
   <213> Künstliche Sequenz
<220>
   <223> Beschreibung der künstlichen Sequenz: HLA-A0201 restringiertes Peptid von p16
<400> 6
   Met Met Gly Ser Ala Arg Val Ala Glu Leu
   1 5 10
<210> 7
   <211> 10
   <212> PRT
   <213> Künstliche Sequenz
<220>
   <223> Beschreibung der künstlichen Sequenz: HLA-A0201 restringiertes Peptid von p16
<400> 7
   Leu Leu Leu His Gly Ala Glu Pro Asn Cys
   1 5 10
<210> 8
   <211> 10
   <212> PRT
   <213> Künstliche Sequenz
<220>
   <223> Beschreibung der künstlichen Sequenz: HLA-A0201 restringiertes Peptid von p16
<400> 8
   Gly Val Met Met Met Gly Ser Ala Arg Val
   1 5 10